# EUROPÄISCHE PATENTSCHRIFT

(11) **EP 1 204 720 B1**
(45) Veröffentlichungstag und Bekanntmachung des Hinweises auf die Patenterteilung: **25.06.2003**
(21) Anmeldenummer: 00936837.4
(22) Anmeldetag: 31.05.2000
(51) Int. Cl.: C10G 11/00, C10G 11/10, C10G 70/04, C07C 4/06

(54) **VERFAHREN ZUM ERZEUGEN VON C2- BIS C4-OLEFINEN AUS EINEM C4 BIS C8-OLEFINE ENTHALTENDEN EINSATZGEMISCH**
METHOD FOR PRODUCING C2 TO C4 OLEFINS FROM A FEED MIXTURE CONTAINING C4 TO C8 OLEFINS
PROCEDE DE PREPARATION D'OLEFINES C2-C4 A PARTIR D'UN MELANGE CONTENANT DES OLEFINES C4-C8

(30) Priorität: 15.07.1999 DE 19933063
(43) Veröffentlichungstag der Anmeldung: 15.05.2002
(73) Patentinhaber: MG Technologies AG, 60325 Frankfurt am Main (DE)
(72) Erfinder: KOSS, Ulrich, D-64291 Darmstadt (DE); ROTHAEMEL, Martin, D-60437 Frankfurt am Main (DE); KÖNIG, Peter, D-61348 Bad Homburg (DE)
(86) Internationale Anmeldenummer: EP0004960
(87) Internationale Veröffentlichungsnummer: WO01005909

(56) Entgegenhaltungen:
- EP-A- 0 490 351
- DE-A- 19 648 795
- US-A- 4 743 357

## Beschreibung

Die Erfindung betrifft ein Verfahren zum Erzeugen von C₂bis C₄-Olefinen aus Wasserdampf und einem C₄- bis C₈-Olefine enthaltenden Einsatzgemisch, wobei man das Wasserdampf enthaltende Einsatzgemisch mit einer Eingangstemperatur von 300 bis 700°C in einen Reaktor leitet, der eine Schüttung aus körnigem, formselektivem Zeolith-Katalysator enthält, wobei man aus der Schüttung ein Wasserdampf und C₂- bis C₄-Olefine enthaltendes Produktgemisch abzieht, welches man durch mindestens einen Kühler leitet.

Ein solches Verfahren ist aus DE 196 48 795 A1 bekannt. Der Erfindung liegt die Aufgabe zugrunde, dieses Verfahren weiterzuentwickeln und dabei möglichst kostengünstig zu arbeiten. Erfindungsgemäß geschieht dies beim eingangs genannten Verfahren dadurch, daß man das aus dem Kühler kommende dampfförmige Produktgemisch verdichtet, wobei der Druck des Produktgemisches um 0,3 bis 7 bar und vorzugsweise mindestens 1 bar erhöht wird, daß man das verdichtete Produktgemisch durch einen indirekten Wärmeaustauscher leitet und darin das Produktgemisch soweit kühlt, daß unter Abgabe von Kondensationswärme ein wasserreiches Kondensat entsteht, daß man das kondensathaltige Produktgemisch in einen Separator leitet, aus welchem man ein wasserreiches Kondensat und, getrennt davon, ein dampfförmiges, C₂- bis C₄-Olefine enthaltendes Produktgemisch abzieht, daß man aus dem Separator kommendes, wasserreiches Kondensat entspannt und unter Ausnutzung der vorher bei der Kondensation abgegebenen Kondensationswärme im indirekten Wärmeaustauscher verdampft, daß man mindestens einen Teil des Wasserdampfs aus dem indirekten Wärmeaustauscher in eine Mischkammer leitet, welcher man das C₄- bis C₈-Olefine enthaltende Einsatzgemisch zuführt und daß man aus der Mischkammer ein Gemisch abzieht, welches man erhitzt und in den Reaktor leitet.

Beim erfindungsgemäßen Verfahren wird durch die Erhöhung des Drucks um 0,3 bis 7 bar und vorzugsweise mindestens 1 bar die Kondensationstemperatur erhöht, so daß schon bei Abkühlung auf die erhöhte Kondensationstemperatur wasserreiches Kondensat anfällt. Das aus dem Separator kommende wasserreiche Kondensat wird um eine Druckdifferenz von 0,3 bis 7 bar entspannt, so daß seine Verdampfungstemperatur unter die Kondensations-temperatur fällt, die zuvor durch Kompression angehoben worden war. Dadurch erreicht man, daß die bei der Kondensation anfallende Wärmemenge direkt wieder zur Verdampfung des wasserreichen Kondensats verwendet werden kann.

Es ist zweckmäßig, wenn das dem Reaktor zugeführte Gemisch aus Wasserdampf und Kohlenwasserstoffen diese Bestandteile im Gewichtsverhältnis von 0,5:1 bis 3:1 enthält. Im Reaktor ist der körnige Zeolith-Katalysator in Form einer Schüttung angeordnet. Die Korngrößen des Katalysators liegen üblicherweise im Bereich von 1 bis 8 mm. Der Zeolith ist vom Pentasil-Typ, er hat formselektive Eigenschaften. Im Katalysator liegt das Atomverhältnis Si:Al im Bereich von 10:1 bis 200:1. Die Primärkristallite des Alumosilikats haben vorzugsweise eine enge Körnungsverteilung mit Durchmessern im Bereich von 0,1 bis 0,9 µm; die BET-Oberfläche liegt üblicherweise im Bereich von 300 bis 600 m²/g, und das Porenvolumen (nach der Quecksilberporosimetrie) beträgt etwa 0,3 bis 0,8 cm³/g. Als Bindemittel zum Zusammenhalten der Primärkristallite wird vorzugsweise Aluminiumoxidhydrat verwendet.

Das zu verarbeitende Einsatzgemisch, welches C₄- bis C₈-Olefine enthält, kann in einem weiten Bereich variieren, z. B. kann es sich um Leichtbenzin aus einer katalytischen Crackanlage oder um ein Raffinat aus dem Produkt eines Spaltofens (Steamcracker) handeln. Das Einsatzgemisch kann auch Kohlenwasserstoffe mit mehr als 8 C-Atomen pro Molekül enthalten, wobei diese höher siedenden Bestandteile vor dem Reaktor vorzugsweise, zumindest zum Teil, entfernt werden. Die Abtrennung muß jedoch nicht vollständig erfolgen, da längerkettige Moleküle für die Umsetzung im Reaktor nicht schädlich sind, sondern vor allem nur überflüssigen Ballast darstellen. Wenn man die längerkettigen Olefine in der Mischkammer abtrennen will, empfiehlt es sich, die Mischkammer als Kolonne mit einer gas- und flüssigkeitsdurchlässigen Packung auszubilden und das Einsatzgemisch auf den oberen Bereich der Packung zu leiten. Gleichzeitig leitet man eine Teilmenge des Wasserdampfs in den unteren Bereich der Packung, wobei diese Teilmenge so ausgewählt wird, daß die C₄- bis C₈-Olefine aus dem Einsatzgemisch verdampfen und zusammen mit dem Wasserdampf aus der Kolonne abgeführt werden. Die höher siedenden Kohlenwasserstoffe bleiben ganz oder weitgehend in der Kolonne und werden aus deren Sumpf zusammen mit gebildetem Wasser abgezogen.

Ausgestaltungsmöglichkeiten des Verfahrens werden mit Hilfe der Zeichnung erläutert. Es zeigt:
- Fig. 1: ein Fließschema des Verfahrens und
- Fig. 2: eine Abwandlung des Verfahrens der Fig. 1.

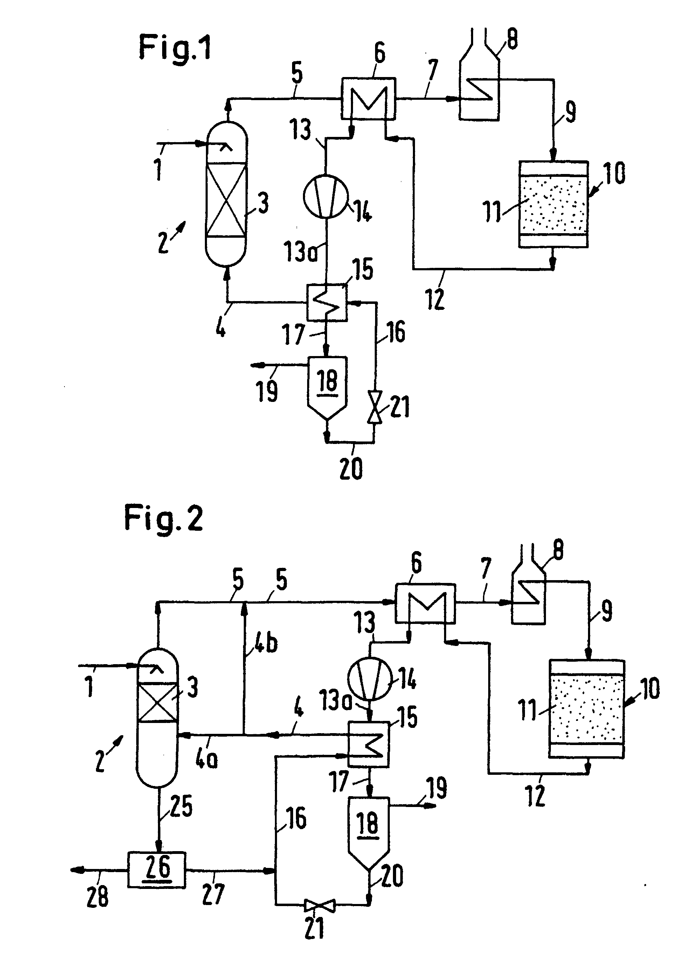

Gemäß Fig. 1 leitet man das C₄- bis C₈-Olefine enthaltende Einsatzgemisch durch die Leitung (1) in die als Mischkammer dienende Kolonne (2), die eine Packung (3) aus gas- und flüssigkeitsdurchlässigen Elementen, z. B. Böden, enthält. Gleichzeitig wird Wasserdampf durch die Leitung (4) herangeführt, der von unten in die Packung (3) eintritt und dabei das Einsatzgemisch verdampft und zum Kopf der Kolonne (2) mitnimmt. Das Gemisch aus Einsatzgemisch und Wasserdampf gelangt durch die Leitung (5) zu einem Wärmeaustauscher (6), in welchem die Temperatur des Gemisches erhöht wird. Schließlich strömt das Gemisch durch die Leitung (7) zu einem Erhitzer (8) , der gefeuert oder elektrisch betrieben werden kann, und verläßt diesen mit einer Temperatur im Bereich von 300 bis 700°C und vorzugsweise 400 bis 600°C. Mit dieser Temperatur wird das Gemisch durch die Leitung (9) in den Reaktor (10) geleitet, der eine Schüttung (11) aus einem formselektiven Zeolith-Katalysator vom Pentasil-Typ enthält. Das Si:Al-Atomverhältnis des Zeolithen liegt im Bereich von 10:1 bis 200:1. Die Zusammensetzung des Einsatzgemisches, welches man durch die Leitung (9) in den Reaktor (10) leitet, kann variieren, dabei empfiehlt es sich, den Aromatengehalt, wasserfrei gerechnet, auf höchstens 20 Gew.-% und vorzugsweise höchstens 10 Gew.-% einzustellen. Dies ist empfehlenswert, weil ein höherer Aromatengehalt durch Kohlenstoffablagerungen zum vorzeitigen Desaktivieren des Katalysators führt. Ferner ist es zweckmäßig, wenn das Einsatzgemisch frei von Komponenten ist, die dreifache C-C-Bindungen oder konjugierte Doppelbindungen haben, da sie ebenfalls den Katalysator desaktivieren.

Die Umsetzung im Reaktor (10) erfolgt adiabatisch, so daß man aus der Schüttung ein Produktgemisch abzieht, dessen Temperatur 20 bis 80°C niedriger als die Eingangstemperatur ist. Der summierte Gehalt an Ethylen, Propylen und Butenisomeren im Produktgemisch, das man in der Leitung (12) abzieht, beträgt mindestens 60 Gew.-% und vorzugsweise mindestens 70 Gew.-% der olefinischen Bestandteile des Einsatzgemisches. Es empfiehlt sich, im Reaktor bei relativ niedrigen Drücken im Bereich von 0,2 bis 3 bar zu arbeiten. Üblicherweise liegen die Drücke im Reaktor im Bereich von 0,6 bis 1,5 bar.

Das Produktgemisch der Leitung (12) gibt einen Teil seiner Wärme im Wärmeaustauscher (6) ab, und es verläßt den Wärmeaustauscher in der Leitung (13) üblicherweise mit einer Temperatur im Bereich von 60 bis 200°C und einem Druck von 0,5 bis 3 bar. Im Verdichter (14) wird der Druck des dampfförmigen Produktgemisches um 0,3 bis 7 bar und zumeist um mindestens 1 bar erhöht, wobei die Temperatur, bei welcher sich Kondensat bildet, ebenfalls erhöht wird. Durch die Leitung (13a) strömt das verdichtete Gemisch zum indirekten Wärmeaustauscher (15). Im Wärmeaustauscher (15) sorgt man für eine intensive Kühlung, wobei wasserreiches Kondensat aus der Leitung (16) als Kühlmedium dient. Dieses Kondensat verdampft dabei, und der gebildete Wasserdampf wird durch die Leitung (4) in die Kolonne (2) geführt. In dem vom Verdichter (14) kommenden Produktgemisch bildet sich durch die Kühlung im indirekten Wärmeaustauscher (15) wasserhaltiges Kondensat. Das Produktgemisch führt man durch die Leitung (17) in einen Abscheider (18) und zieht daraus durch die Leitung (19)
das gewünschte C₂- bis C₄-Olefine enthaltende Produkt ab, welches noch in eine nicht dargestellte Nachreinigung gegeben werden kann. Das im Separator (18) anfallende wasserreiche Kondensat gelangt durch die Leitung (20) zunächst zu einem Entspannungsventil (21), wo es um eine Druckdifferenz von 0,3 bis 7 bar entspannt wird. Dabei kühlt sich das Kondensat weiter ab und seine Verdampfungstemperatur sinkt. Die weitere Nutzung dieses Kondensats über die Leitung (16) wurde bereits erläutert.

Die Verfahrensvariante der Fig. 2 betrifft die Verarbeitung eines in der Leitung (1) herangeführten Einsatzgemisches, welches neben C₄- bis C₈-Olefinen auch höher siedende Bestandteile enthält. Soweit in Fig. 2 die gleichen Bezugsziffern wie in Fig. 1 verwendet werden, haben diese die bereits zusammen mit Fig. 1 erläuterte Bedeutung. Aus dem indirekten Wärmeaustauscher (15) zieht man in der Leitung (4) Wasserdampf ab, den man auf die Leitungen (4a) und (4b) aufteilt. Die in der Leitung (4a) strömende Wasserdampfmenge reicht aus, um die in der Leitung (1) herangeführten C₄- bis C₈-Olefine in der Kolonne (2) zu verdampfen, wobei aber die höher siedenden Kohlenwasserstoffe weitgehend nicht verdampfen und sich als Flüssigkeit, zusammen mit Wasser, im Sumpf der Kolonne (2) sammeln. Von da aus wird das Flüssigkeitsgemisch durch die Leitung (25) zu einem Abscheider (26) geführt, aus welchem man das abgeschiedene Wasser durch die Leitung (27) dem Kondensat der Leitung (16) zugibt. Die abgeschiedenen Kohlenwasserstoffe werden in der Leitung (28) aus dem Verfahren entfernt. Dem Gemisch aus Wasserdampf und C₂- bis C₄-Olefinen, das man in der Leitung (5) aus der Kolonne (2) abzieht, gibt man den zweiten Wasserdampf-Teilstrom zu, der in der Leitung (4b) abgezweigt wurde und führt das Gemisch zunächst zum Wärmeaustauscher (6), bevor die, zusammen mit Fig. 1, erläuterte Weiterbehandlung stattfindet.

### Beispiele:

Es wird wie in der Zeichnung dargestellt gearbeitet, wobei der Zeolith-Katalysator vom Pentasiltyp ein Si: Al-Atomverhältnis von 70 aufweist. Das Einsatzgemisch des Beispiels 1 weist nur Kohlenwasserstoffe bis C₈ auf, im Beispiel 2 werden auch höhere Kohlenwasserstoffe verarbeitet.

### Beispiel 1:

Dem Verfahren gemäß Fig. 1 werden 100 000 kg/h eines Einsatzgemisches zugeführt, dessen Zusammensetzung in Tabelle 1 angegeben ist und das eine Temperatur von 80° C hat:

**Tabelle 1:**

| | **Beisp.** **1** | **Beisp.** **2** | **A** | **B** |
|---|---|---|---|---|
| Nichtzyklische C₄ bis C₈- Olefine (Gew.%) | 48,0 | 27,0 | 0,2 | 33,0 |
| Nichtzyklische C₈₊ -Olefine(Gew. %) | -- | 4,0 | 6,8 | 3,0 |
| C₄ - bis C₈ - Paraffine (Gew.%) | 38,0 | 19,0 | 0,2 | 23,3 |
| C₈₊ - Paraffine (Gew.%) | -- | 5,0 | 9,4 | 3,9 |
| Aromaten (bis C₈) (Gew. %) | 8,0 | 13,0 | 0,6 | 14,7 |
| Aromaten ( C₈₊) (Gew. %) | -- | 11,0 | 51,7 | 3,2 |
| Zykloalkane und Zykloalkene bis C₈ (Gew.%) | 6,0 | 14,0 | 4,3 | 16,3 |
| Zykloalkane, Zykloalkene, Polynaphthene, C₈₊ (Gew.%) | -- | 7,0 | 26,8 | 2,6 |

Unter Zufuhr von 150000 kg/h Wasserdampf aus der Leitung (4) wird das Einsatzgemisch in der Kolonne (2) vollständig verdampft und nach oben aus der Kolonne (2) geführt. Die Temperatur und der Druck in verschiedenen Leitungen ist in der Tabelle 2 angesehen

**Tabelle 2:**

| | **Beispiel 1** | | **Beispiel 2** | |
|---|---|---|---|---|
| Leitung | Temperatur(°C) | Druck(bar) | Temperatur(°C) | Druck(bar) |
| 5 | 111 | 1,7 | 111 | 1,7 |
| 7 | 420 | 1,5 | 420 | 1,5 |
| 9 | 490 | 1,4 | 490 | 1,4 |
| 12 | 440 | 1,2 | 440 | 1,2 |
| 13 | 170 | 1,1 | 170 | 1,1 |
| 13 a | 145 | 4,2 | 145 | 4,2 |
| 17 | 121 | 4,0 | 121 | 4,0 |
| 19 | 121 | 4,0 | 121 | 4,0 |
| 16 | 117 | 1,8 | 117 | 1,8 |
| 4 | 117 | 1,75 | 117 | 1,75 |
| 25 | - - | - - | 114 | 1,8 |

Die Leistung des Verdichters (14) beträgt 17 MW, zur direkten Kühlung wird zwischen den Verdichterstufen Wasser in das Gemisch eingedüst. Das aus der Leitung (19) abgezogene Produktgemisch, dessen Kohlenwasserstoffe noch dampfförmig sind, hat die in Tabelle 3 angegebene Zusammensetzung:

**Tabelle 3:**

| | **Beispiel 1** | **Beispiel 2** |
|---|---|---|
| Propylen (Gew. %) | 16,3 | 12,8 |
| Ethylen (Gew. %) | 3,7 | 3,0 |
| Butene (Gew. %) | 12,2 | 9,4 |
| Olefine, C₄ bis C₈ (Gew. %) | 2,4 | 5,4 |
| Andere (Gew. %) | 44,4 | 48,4 |
| Wasserdampf (Gew. %) | 21,0 | 21,0 |

Die Verdichtung der Reaktionsprodukte macht es also möglich, daß 95 MW Wärme aufgebracht und eine Kühlleistung von ebenfalls etwa 95 MW, die zum Auskondensieren des Wassergehalts im Produktgemisch erforderlich ist, dadurch eingespart werden, daß man 17 MW Verdichterleistung aufbringt.

### Beispiel 2:

Man arbeitet gemäß Fig. 2 und führt der Kolonne (2) durch die Leitung (1) 100000 kg/h eines Einsatzgemisches mit 80°C und der in Tabelle 1 (oben) angegebenen Zusammensetzung zu. In diesem Einsatzgemisch ist ein höherer Anteil an schwersiedenden Komponenten als im Einsatzgemisch des Beispiels 1 enthalten. Durch die Leitung (4a) werden 33 000 kg/h Prozeßdampf zugeführt, wobei 83 Gew. % des Einsatzgemisches verdampft und nach oben aus der Kolonne(2) abgeführt werden. Der nichtverdampfte Rest des Einsatzgemisches wird mit wäßrigem Kondensat durch die Leitung (25) abgezogen. Die in der Leitung (28) abgezogenen Kohlenwasserstoffe (16970 kg/h) haben die in Tabelle 1, Spalte A angegebene Zusammensetzung. Das durch Leitung (27) abgezogene Kondensat wird in die Leitung (16) eingespeist und so in den Kondensatkreislauf zurückgeführt. Der Kohlenwasserstoffanteil des Gemisches, das die Kolonne (2) über Kopf verläßt, hat die in Tabelle 1, Spalte B angegebene Zusammensetzung. Die folgenden Prozeßschritte sind analog zu den in Beispiel 1 beschriebenen. Die Temperatur und der Druck in den verschiedenen Leitungen ist in Tabelle 2 angegeben. Diesem Gemisch werden durch die Leitung (4b) noch 100000 kg/h Wasserdampf zugemischt, so daß der im Reaktoreinsatzstrom erforderliche Wasserdampfgehalt erreicht wird.

## Patentansprüche

1. Verfahren zum Erzeugen von C₂- bis C₄-Olefinen aus Wasserdampf und einem C₄- bis C₈-Olefine enthaltenden Einsatzgemisch, wobei man das Wasserdampf enthaltende Einsatzgemisch mit einer Eingangstemperatur von 300 bis 700°C in einen Reaktor leitet, der eine Schüttung aus körnigem, formselektivem Zeolith-Katalysator enthält, wobei man aus der Schüttung ein Wasserdampf und C₂- bis C₄-Olefine enthaltendes Produktgemisch abzieht, welches man durch mindestens einen Kühler leitet, **dadurch gekennzeichnet, daß** man das aus dem Kühler ( 6) kommende dampfförmige Produktgemisch verdichtet, wobei der Druck des Produktgemisches um 0,3 bis 7 bar erhöht wird, daß man das verdichtete Produktgemisch durch einen indirekten Wärmeaustauscher ( 15 ) leitet und darin das Produktgemisch soweit kühlt, daß unter Abgabe von Kondensationswärme ein wasserreiches Kondensat entsteht, daß man das kondensathaltige Produktgemisch in einen Separator leitet, aus welchem man ein wasserreiches Kondensat und, getrennt davon, ein dampfförmiges, C₂- bis C₄-Olefine enthaltendes Produktgemisch abzieht, daß man aus dem Separator kommendes, wasserreiches Kondensat entspannt und unter Ausnutzung der vorher bei der Kondensation abgegebenen Kondensationswärme im indirekten Wärmeaustauscher verdampft, daß man mindestens einen Teil des Wasserdampfs aus dem indirekten Wärmeaustauscher in eine Mischkammer leitet, welcher man das C₄- bis C₈-Olefine enthaltende Einsatzgemisch zuführt, und daß man aus der Mischkammer ein wasserdampfhaltiges Gemisch abzieht, welches man erhitzt und in den Reaktor leitet.

2. Verfahren nach Anspruch 1, **dadurch gekennzeichnet, daß** das dem Reaktor zugeführte Einsatzgemisch Wasserdampf und Kohlenwasserstoffe im Gewichtsverhältnis von 0,5:1 bis 3:1 enthält.

3. Verfahren nach Anspruch 1 oder 2, **dadurch gekennzeichnet, daß** das Produktgemisch, das aus dem Kühler ( 6 ) kommt, eine Temperatur von 60 bis 200°C und einen Druck von 0,5 bis 3 bar aufweist und noch dampfförmig ist.

4. Verfahren nach Anspruch 1 oder einem der folgenden, **dadurch gekennzeichnet, daß** das der Mischkammer zugeführte Einsatzgemisch Kohlenwasserstoffe mit mehr als 8 C-Atomen pro Molekül enthält, daß die Mischkammer als Kolonne mit einer gas- und flüssigkeitsdurchlässigen Packung ausgebildet ist, daß man das Einsatzgemisch auf den oberen Bereich der Packung leitet und mit einer der Kolonne in den unteren Bereich der Packung zugeführten Teilmenge des Wasserdampfs C₄- bis C₈-Olefine aus dem Einsatzgemisch verdampft und zusammen mit dem Wasserdampf aus der Kolonne abführt.

5. Verfahren nach Anspruch 4, **dadurch gekennzeichnet, daß** man aus der Kolonne ein Wasser und Kohlenwasserstoffe enthaltendes Flüssigkeitsgemisch abzieht, aus welchem man Wasser abtrennt, welches man dem entspannten wasserreichen Kondensat vor dem indirekte Wärmeaustauscher zugibt.

## Claims

1. A process for producing C₂ to C₄ olefins from steam and a feed mixture containing C₄ to C₈ olefins, wherein the steam-containing feed mixture is passed at an entry temperature of 300 to 700°C into a reactor which contains a bed of granular, form-selective zeolite catalyst, wherein a product mixture containing steam and C₂ to C₄ olefins is withdrawn from the bed and is passed through at least one cooler, **characterised in that** the vaporous product mixture coming from the cooler (6) is compressed, the pressure of the product mixture being increased by 0.3 to 7 bar, that the compressed product mixture is passed through an indirect heat exchanger (15) and the product mixture is cooled therein until a water-rich condensate is produced, with heat of condensation being giving off, that the condensate-containing product mixture is passed into a separator from which a water-rich condensate and, separately therefrom, a vaporous product mixture containing C₂ to C₄ olefins is withdrawn, that water-rich condensate coming from the separator is expanded and, utilising the heat of condensation previously given off in the condensation, is evaporated in the indirect heat exchanger, that at least part of the steam from the indirect heat exchanger is passed into a mixing chamber to which the feed mixture containing C₄ to C₈ olefins is supplied, and that a steam-containing mixture is withdrawn from the mixing chamber, which mixture is heated and passed into the reactor.

2. A process according to Claim 1, **characterised in that** the feed mixture supplied to the reactor contains steam and hydrocarbons in a weight ratio of 0.5:1 to 3:1.

3. A process according to Claim 1 or 2, **characterised in that** the product mixture which comes from the cooler (6) is at a temperature from 60 to 200°C and a pressure of 0.5 to 3 bar and is still in vapour form.

4. A process according to Claim 1 or one of the following claims, **characterised in that** the feed mixture supplied to the mixing chamber contains hydrocarbons having more than 8 C atoms per molecule, that the mixing chamber is in the form of a column with a gas- and liquid-permeable packing, that the feed mixture is passed on to the upper region of the packing and C₄ to C₈ olefins from the feed mixture are evaporated with a partial quantity of the steam supplied to the column in the lower region of the packing and are withdrawn from the column together with the steam.

5. A process according to Claim 4, **characterised in that** a liquid mixture containing water and hydrocarbons is withdrawn from the column, from which mixture water is separated off which is supplied to the expanded water-rich condensate before the indirect heat exchanger.

## Revendications

1. Procédé de préparation d'oléfines ayant de 2 à 4 atomes de carbone à partir de vapeur d'eau et d'un mélange de charge contenant des oléfines ayant de 4 à 8 atomes de carbone, en envoyant le mélange de charge contenant de la vapeur d'eau et ayant une température d'entrée de 300 à 700°C dans un réacteur, qui contient un tas de catalyseur sous forme de zéolite en grains à sélectivité de forme, en soutirant du tas un mélange de produits contenant de la vapeur d'eau et des oléfines en C₂ à C₄ que l'on envoie dans au moins un dispositif de refroidissement, **caractérisé en ce que** l'on comprime le mélange de produits sous forme de vapeur venant du dispositif (6) de refroidissement, la pression du mélange produit étant augmentée de 0,3 à 7 bar, **en ce que** l'on envoie le mélange produit comprimé dans un échangeur de chaleur (15) par voie indirecte et que l'on y refroidit ainsi le mélange produit jusqu'à ce qu'il se forme, en cédant de la chaleur de condensation, un produit condensé riche en eau, **en ce que** l'on envoie le mélange produit contenant du produit condensé dans un séparateur dont on soutire un produit condensé riche en eau et, indépendamment de cela, un mélange produit sous forme de vapeur contenant des oléfines ayant de 2 à 4 atomes de carbone, **en ce que** l'on détend le produit condensé riche en eau provenant du séparateur et on l'évapore en tirant parti de la vapeur de condensation cédée précédemment lors de la condensation dans un échangeur de chaleur par voie indirecte, **en ce que** l'on envoie au moins une partie de la vapeur d'eau de l'échangeur de chaleur indirect dans une chambre de mélange à laquelle on envoie le mélange de charge contenant des oléfines ayant de 4 à 8 atomes de carbone et **en ce que** l'on soutire de la chambre de mélange un mélange contenant de la vapeur d'eau que l'on chauffe et que l'on envoie au réacteur.

2. Procédé suivant la revendication 1, **caractérisé en ce que** le mélange de charge envoyé au réacteur contient de la vapeur d'eau et des hydrocarbures dans un rapport en poids de 0,5:1 à 3:1.

3. Procédé suivant la revendication 1 ou 2, **caractérisé en ce que** le mélange produit qui vient du dispositif (6) de refroidissement a une température de 60 à 200°C et une pression de 0,5 à 3 bar et est encore sous forme de vapeur.

4. Procédé suivant la revendication 1 ou l'une des suivantes, **caractérisé en ce que** le mélange de charge envoyé à la chambre de mélange contient des hydrocarbures ayant plus de 8 atomes de carbone par molécule, **en ce que** la chambre de mélange est constituée sous la forme d'une colonne ayant un garnissage perméable aux gaz et aux liquides, **en ce que** l'on envoie le mélange de charge sur la partie supérieure du garnissage et **en ce que** l'on évapore, avec une quantité partielle de la vapeur d'eau envoyée à la colonne dans la partie inférieure du garnissage, des oléfines ayant de 4 à 8 atomes de carbone du mélange de charge et on les évacue de la colonne ensemble avec la vapeur d'eau.

5. Procédé suivant la revendication 4, **caractérisé en ce que** l'on soutire de la colonne un mélange liquide contenant de l'eau et des hydrocarbures, dont on sépare l'eau que l'on ajoute avant l'échangeur de chaleur par voie indirecte au produit condensé détendu riche en eau.
